# EUROPEAN PATENT APPLICATION

(11) **EP 0 717 283 A2**
(43) Date of publication of application: **19.06.1996**
(21) Application number: 95309024.8
(22) Date of filing: 12.12.1995
(51) Int. Cl.: G01N 33/52, G01N 33/569, C12Q 1/70

(54) **Device and test kit for the detection of AIDS**

(30) Priority: 13.12.1994 US 32042
(71) Applicant: ORTHO PHARMACEUTICAL CORPORATION, Raritan, NJ 08869 (US)
(72) Inventor: Millenson, Elliot J., Far Hills, New Jersey 07931 (US)
(74) Representative: Fisher, Adrian John

(57) **Abstract**

Disclosed herein is a diagnostic instrument designed for use with an in-home test kit to be used by a consumer to test for the presence of the HIV virus (human immunodeficiency virus) which is known to cause AIDS (Acquired Immune Deficiency Syndrome).

## Description

### Field of the Invention

The present invention relates generally to a diagnostic instrument for use in detecting a foreign element in a blood sample. More particularly, the present invention relates to a diagnostic instrument designed for use with an in-home specimen collection kit to be used by a consumer to test for the presence of the HIV virus (human immunodeficiency virus) which is known to cause AIDS (Acquired Immune Deficiency Syndrome).

### Background of the Invention

What is viewed by many as the single most serious modern-day health issue affecting all members of society is the life-threatening risk posed by AIDS. Since there is presently no known cure for the disease, early detection and treatment of the HIV virus are the best chances of helping a patient prolong his or her life by delaying onset of the disease while preventing the spread of the virus itself.

Although early detection of the HIV virus is vitally important, only about 8% of adult Americans are tested each year. Unfortunately, individuals have been reluctant to submit to testing. One of the most significant reasons for this reluctance is the fear that a positive test result will not be kept confidential. It is currently estimated that the number of at-risk individuals being tested for the HIV virus would increase to about 29% if a diagnostic procedure was available that could assure the individual's confidentiality.

Briggs et al. have attempted to provide an at-home testing system for determining whether an individual may be carrying the HIV virus. The system is disclosed in U.S. Patent Numbers 5,190,049, 4,979,515 and 4,777,964 which cover methods and devices by which an individual collects his or her own blood specimen and submits the specimen for subsequent analysis of bacterial, viral or chemical components. According to the Briggs et al. system, blood specimens are placed into glass vials which must be carefully sealed before being forwarded for laboratory evaluation. The blood collection device of Briggs et al. is cumbersome and requires a consumer to not only approximate the quantity of blood being collected, but also to seal the vial with clay or putty before transporting it.

What is lacking in the art is a device by which an individual may conveniently collect a blood sample that can be submitted for diagnostic evaluation. The device should allow the individual to collect an accurate amount of blood to be tested and should be suitable for transporting through routine channels. The present invention solves these problems.

### Summary of the Invention:

The present invention provides a collection device utilized by a lay individual for that individual to collect his or her own whole blood specimen and submit the specimen to a laboratory for subsequent analysis, wherein the specimen is absorbed into the device and wherein the device comprises a front and back planar surface, the surfaces being parallel to each other; a cut-out portion associated with the surfaces; and a test strip forming a laminate on the device.

The present invention also provides a kit utilized by a lay individual for that individual to collect his or her own whole blood specimen and submit the specimen to a laboratory for subsequent analysis comprising (a) instructional booklets; (b) antiseptic wipes; (c) rupturing means for rupturing the dermis layer of the skin of the individual; (d) means for collecting and disposing of the rupturing means; (e) means for bandaging the individual's ruptured dermis layer; (f) a whole blood collection device comprising a front and back planar surface, the surfaces being parallel to each other; a cut-out portion associated with the surfaces; and a test strip forming a laminate on the device; and (g) means for transporting the collected whole blood specimen to a laboratory for subsequent analysis.

Once a dried whole blood specimen has been placed on the present collection device, the device is simply inserted into a mailer and is forwarded to a diagnostic center through routine postal services.

According to one embodiment of the present invention, there is provided a CONFIDE™ card, which is part of the CONFIDE™ HIV testing service, that allows individuals to collect their own whole blood specimen which may contain specific analytes for HIV, then submit such samples to laboratory testing services for HIV analysis, obtain test results of the samples and receive medical counseling, with complete privacy assured. Examples of such analytes are HIV antigen, antibodies specific for HIV, and nucleic acid sequences specific for HIV.

The CONFIDE™ card enables individuals to easily and privately self-collect a blood specimen. User anonymity is safeguarded by the 14-digit code number found on each CONFIDE™ card which individuals use to identify themselves when they phone for testing results, customer service or counseling.

In a preferred embodiment of the present invention, the CONFIDE™ card consists of a test strip comprising filter paper, which is laminated between two layers of coated paperboard, with a window or cut-out area exposing the front and back of the test strip. Three circles measuring about 5/16 inch diameter are printed on the front of the test strip window. Three circles measuring about 1/4 inch diameter are printed on the back, concentric within each front circle. The CONFIDE™ card is printed with illustrations and instructions directing the user to completely fill each circle with blood until the blood soaks through to the other side of the card. Smaller concentric circles are provided on the back of the card as quality control indicators to allow the user to determine if he or she has properly obtained an adequate blood sample for testing purposes. Since an acceptable sample size would preferably fill the smaller three 3/4 inch circles, by instructing a user to fill the three larger 5/16 inch circles on the front of the card with his or her whole blood specimen assures the three 3/4 inch circles on the back of the card will be completely filled, thus facilitating further analysis.

The CONFIDE™ card also has a lower tear-off portion. A unique Personal Identification Number (PIN) is printed on both the top and bottom portions of the card. Users mail the upper portion of the card with their dried whole blood specimen and retain the lower portion of the card containing their PIN. The bottom portion of the CONFIDE™ card also contains a telephone number to be utilized by an individual to obtain his or her test results.

The filter paper of the test strip conforms to the specifications set by the National Committee for Clinical Laboratory Standards entitled Blood Collection on Filter Paper for Neonatal Screening Programs, NCCLS Document LA4-A2, ISBN 1-56238-157-1, ISSN 0273-3099, Volume 12 Number 13, July 1992. The specifications are as follows:
1. Filter paper should be made of 100% pure cotton fiber, with no wet-strength additives.
2. Basis weight should be 110 lb ± 5% per ream. A ream is defined as 500 sheets 24" x 36" (ASTM D-646-67).
3. Densometer: Densometer reading is taken by the Gurley method of one sheet with a 5-oz cylinder, 0.1 in² orifice and 100 cc of air (test method, modified ASTM D726-58)
4. pH should be 5.7 to 7.5.
5. Ash %: 0.1% maximum (Test method, modified ASTM D586-63).
6. Klemm: Tappi modified useful method - UM451.
7. Wet strength: (ASTM D-774-67), usually around 4.5 lbs/in².
8. Absorbance rate: The absorption time and the diameter of blood spot produced by 100 µL of a fresh whole blood sample (hematocrit 55 ± 1%) should be measured. Absorption time target: 12 sec; range 5 to 30 sec. Diameter target: 16 mm; report <15 mm and >17 mm volume. Ragged edges or mottling of dried blood spot should be recorded if observed.

The assigned PIN must be provided by a user upon calling to obtain his or her test results. The PIN is in machine readable form which is bar coded on the top portion of the card so that it can be scanned at the testing laboratory. When the card is processed, the laboratory will verify the PIN on the card is valid by cross-referencing the number against a master list of valid PINs provided to the testing laboratory.

### Brief Description of Drawings

- FIG. 1: is a perspective view of the card of the present invention;
- FIG. 2: is a cross-sectional view of the blood sample region taken along line 2-2 of FIG. 1;
- FIG. 3: is a rear elevational view of the card of FIG. 1 shown with identification card in a detached position;
- FIG. 4: is a perspective view of a kit for which the card of the present invention may be used;
- FIG. 5: is a flow chart disclosing the various steps to perform the method of use for the card of the present invention.

### Detailed Description of the Drawings

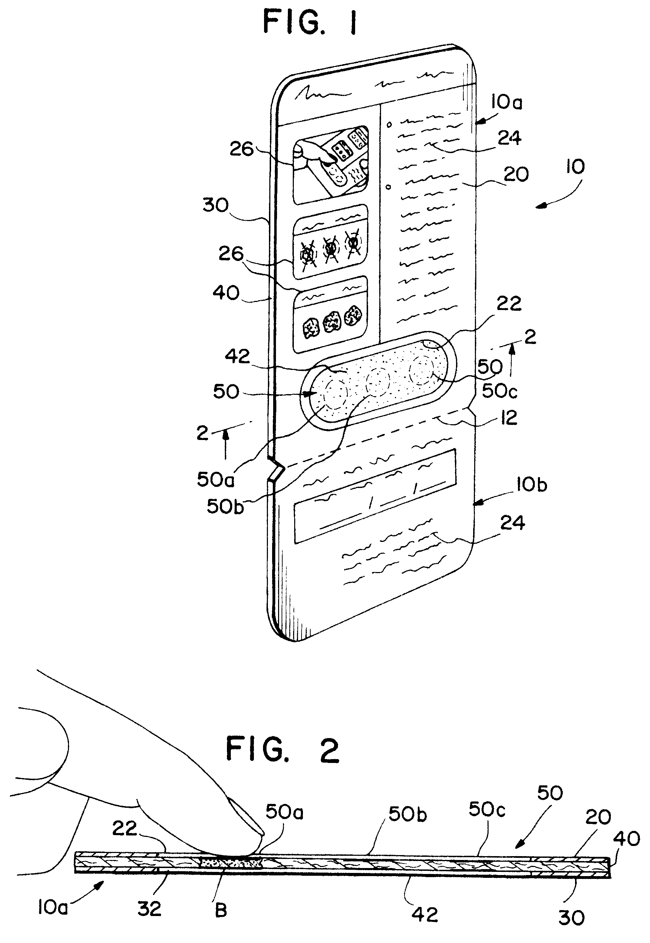

With reference to Figures 1, 2, and 3, disclosed is the card of the present invention shown generally as 10.

Card 10 is intended to be used as a simple means for a user to apply his or her blood sample to one portion of the card, detach the identification portion and return the test portion to a testing laboratory for evaluation of the blood sample. The method of use will be described in greater detail hereinafter.

Card 10 is generally rectangular in shape and is comprised of paper layer 20 and paper layer 30 laminated to a centrally disposed absorbent layer 40. Additionally, card 10 contains a perforation 12 which allows card 10 to be separated into test portion 10a and user identification portion 10b.

Paper layer 20 and paper layer 30 may be affixed to absorbent layer 40 by using conventional means such as heat sealing, adhesives and the like. Located in layers 20 and 30 are cut out regions 22 and 32 respectively, which are in alignment with each other. Cut out regions 22 and 32 define an exposed region of absorbent layer 40 to create sample window 50.

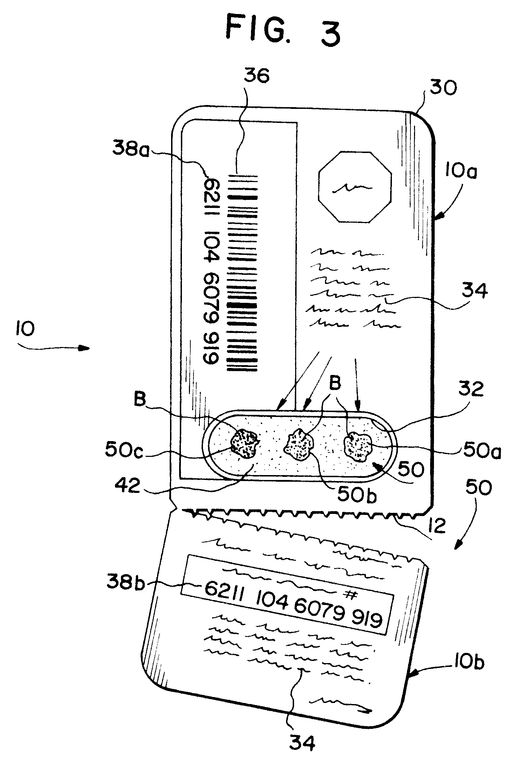

Located within blood sample window 50 are three zones, 50a, 50b and 50c, which are printed on both sides of absorbent layer 40. The purpose of having zones 50a, 50b and 50c printed on both sides of absorbent layer 40 is to allow the user to ensure that the blood sample is thorough and complete since it must soak or migrate through the entire thickness of layer 40 to an area which exceeds the area defined by zones 50a, 50b and 50c. This can best be seen in FIG. 3 which shows zones 50a, 50b and 50c with blood samples B migrating through the entire thickness of absorbent layer 40.

Paper layers 20 and 30 can be made from a variety of different materials including conventional bond paper, coated paper stock and plastic film. Absorbent layer 40 can be made from a variety of conventional materials which would be known by a skilled artisan.

Paper layer 20 has printed indicia on its outer surface comprising written text 24 and pictorial representation 26 which may be used for instructional purposes. Paper layer 30 has printed indicia on its outer surface comprising written text 34, bar code 36 and identification numbers 38a and 38b.

Identification numbers 38a and 38b are identical. This allows the test result center to use identification number 38a together with bar code 36 to positively identify the test samples thus ensuring test results are accurately dispensed.

Identification number 38b allows a user to access his or her test results when contacting the test result center.

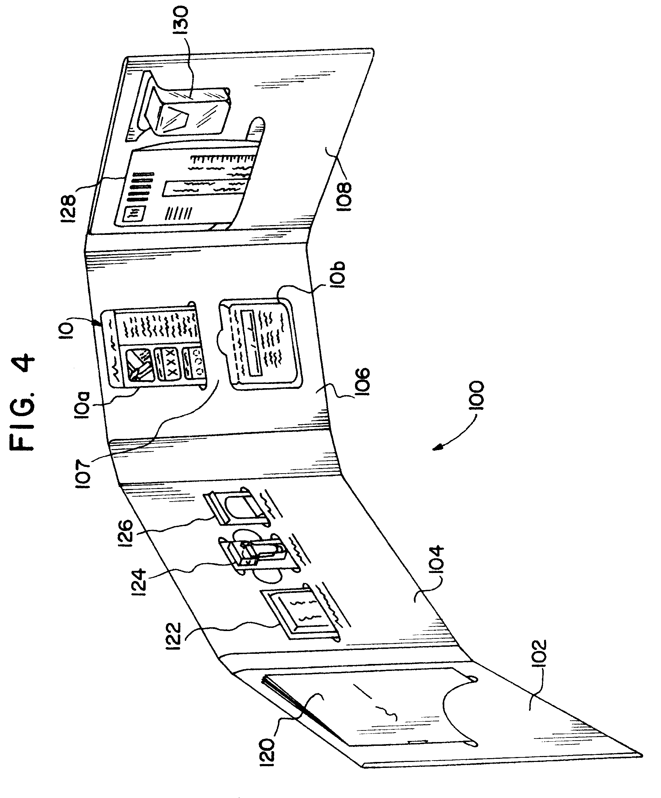

Now referring to FIG. 4, a CONFIDE™ HIV kit 100 is shown in an open position, ready for use with the card 10 of the present invention.

Kit 100 is a four part folder comprised of sections 102, 104, 106 and 108. Contained in folder 102 is instructional booklet 120. Located in folder portion 104 is antiseptic 122, lancet 124 and bandage 126. Positioned in folder portion 106 is card 10. A portion 107 of folder 106 is disposed adjacent to the test region 50 of the card 10 to protect it from contamination until the test is conducted. Located in folder portion 108 is mailer 128 and lancet disposal container 130. Kit 100 is designed so that the user can sequentially perform the tasks necessary to complete the blood test.

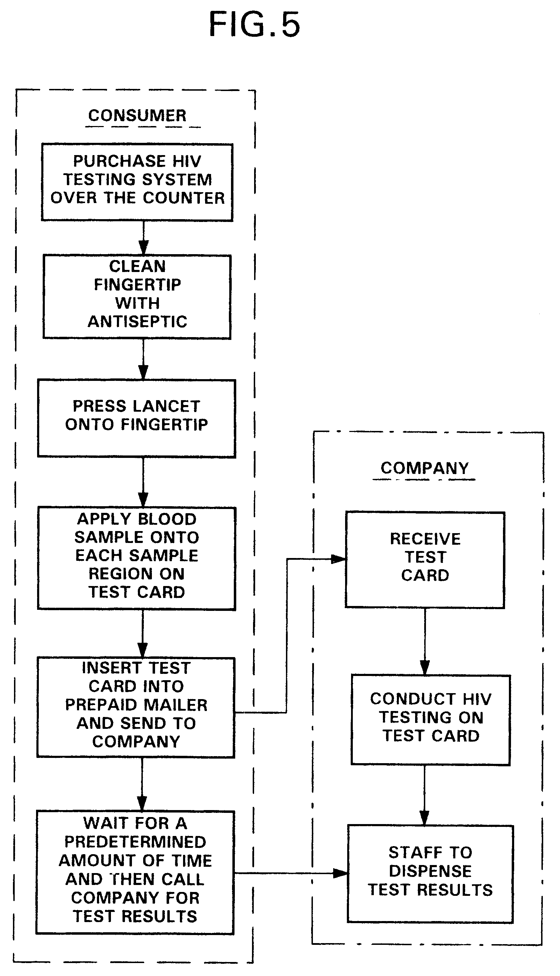

The sequence of steps used to complete the blood test is shown in FIG. 5. After purchasing the kit and reviewing the instructions, a user would wipe a finger with antiseptic 122 to clean the surface of his or her fingertip and then press lancet 124 into the fingertip thereby causing a small laceration from which droplets of blood can then be applied to each of the zones in the test region 50 of the card 10. After the blood has dried, the user simply places the upper detached portion 10a into mailer 128 and deposits the mailer in the mail. After approximately seven days from the date of mailing, the user can telephone the test result center and obtain the test results by furnishing his or her identification number to the representative of the center.

A variety of further modifications to the card described herein are believed to be apparent to those skilled in the art. Thus, it is intended that the present invention cover such modifications, provided they come within the scope of the claims of this invention and their equivalents.

## Claims

1. A device utilized by a lay individual for that individual to collect his or her own whole blood specimen and submit said specimen to a laboratory for subsequent analysis, wherein said specimen is absorbed into said device and wherein said device comprises a front and back planar surface, said surfaces being parallel to each other; a cut-out portion associated with said surfaces; and a test strip forming a laminate on said card.

2. The device according to claim 1 wherein said whole blood specimen is analyzed for antibody for HIV.

3. The device according to claim 1 wherein said whole blood specimen is analyzed for antigen for HIV.

4. The device according to claim 1 wherein said whole blood specimen is analyzed for nucleic acid for HIV.

5. The device according to claim 1 wherein said cut-out portion comprises a plurality of concentric openings, said openings being linearly spaced apart from each other and having a larger diameter on said front planar surface and a smaller diameter on said back planar surface.

6. The device according to claim 1 wherein instructions are printed on said planar surfaces.

7. The device according to claim 6 further comprising detachable top and bottom portions, said portions having matching PINs.

8. The device according to claim 7 wherein said PINs are in machine-readable form.

9. A kit utilized by a lay individual for that individual to collect his or her own whole blood specimen and submit said specimen to a laboratory for subsequent analysis comprising (a) instructional booklets; (b) antiseptic wipes; (c) rupturing means for rupturing the dermis layer of the skin of the individual; (d) means for disposing said rupturing means; (e) means for bandaging the individual's ruptured dermis layer; (f) a whole blood collection device further comprising a front and back planar surface, said surfaces being parallel to each other; a cut-out portion associated with said surfaces; and a test strip forming a laminate on said device; and (g) means for transporting said collected whole blood specimen to a laboratory for subsequent analysis.

10. The kit according to claim 9 wherein said whole blood specimen is analyzed for antibody for HIV.

11. The kit according to claim 9 wherein said whole blood specimen is analyzed for antigen for HIV.

12. The kit according to claim 9 wherein said whole blood specimen is analyzed for nucleic acid for HIV.

13. The kit according to claim 10 wherein said cut-out portion comprises a plurality of concentric openings having the same diameters, said openings being linearly spaced apart from each other.

14. The kit according to claim 9 having instructions printed on said planar surfaces.

15. The kit according to claim 14 further comprising detachable top and bottom portions, said portions having matching PINs.

16. The kit according to claim 15 wherein said PINs are in machine-readable form.
